# EUROPEAN PATENT SPECIFICATION

(11) **EP 0 750 912 B1**
(45) Date of publication and mention of the grant of the patent: **29.03.2000**
(21) Application number: 96110400.7
(22) Date of filing: 27.06.1996
(51) Int. Cl.: A61K 38/48, A61K 38/58, C07K 14/46

(54) **Use of batroxobin containing medicaments for the treatment or prophylaxis of ischemia-reperfusion injury**
Verwendung von Batroxobin enthaltende Arzneimittel zur Behandlung und Prophylaxe von ischämischen Reperfusionsschäden
Utilisation des médicaments contenant de la batroxobine pour le traitement et al prophylaxie des lésions consécutives à des perfusions répétées en cas d'ischémie

(30) Priority: 28.06.1995 JP 16166595
(43) Date of publication of application: 02.01.1997
(73) Proprietor: TOBISHI PHARMACEUTICAL CO., LTD., Chiyoda-ku, Tokyo (JP)
(72) Inventor: Tomaru, Takanobu, Nerima-ku, Tokyo (JP); Kuang, Pei-Gen, HaiDian District, Beijing 100853 (CN)
(74) Representative: VOSSIUS & PARTNER

(56) References cited:
- EP-A- 0 719 791
- FR-A- 2 578 745
- US-A- 3 849 252
- TANAHASHI N. ET AL: "Effect of single intravenous administration of batroxobin on erythrocyte aggregability in patients with acute-stage cerebral infarction" CLINICAL HEMORHEOLOGY, vol. 15, no. 1, 1995, USA, pages 89-96, XP000197385
- GORDON D.O. LOWE: "Defibrination, blood flow and blood rheology" CLINICAL HEMORHEOLOGY, vol. 4, 1984, USA, pages 15-28, XP000195919
- LATALLO Z S: "INTERNATIONAL COMMITTEE COMMUNICATIONS. RETROSPECTIVE STUDY ON COMPLICATIONS AND ADVERSE EFFECTS OF TREATMENT WITH THROMBIN-LIKE ENZYMES - A MULTICENTRE TRIAL" THROMBOSIS AND HAEMOSTASIS, vol. 50, no. 2, 1983, pages 604-609, XP000197388
- WITZKE-GROSS J: "PERIPHERE UND ZEREBRALE ARTERIELLE DURCHBLUTUNGSSTOERUNGEN" DEUTSCHE APOTHEKER ZEITUNG, vol. 130, no. 25, 21 June 1990, pages 187-193, XP000197387
- DATABASE CHEMABS CHEMICAL ABSTRACTS SERVICE, COLUMBUS, OHIO, US AN: 117:205106, NAMIKATA, SHOHEI ET AL: "Effects of snake venom batroxobin (defibrase) on cerebral infarction in a rat model" XP002045039

## Description

The present invention relates to use of batroxobin for the preparation of a medicament for the treatment or prophylaxis of ischemia-reperfusion injury.

Ischemia-reperfusion injury is a general term for an injury which occurs after blood circulation is restarted in a tissue of an ischemic organ when an excision operation or ablation of various organs is conducted. Such injury also occurs during transplantation of an organ with a blood vessel when the blood circulation is restarted after it is stopped. Thus, such injury frequently occurs in many tissues, such as kidney, liver, lungs, pancreas and digestive tubes as well as heart and brain.

So far, it has been assumed that ischemia-reperfusion injury is caused by a rise in energy metabolism or an increase in an active oxygen species by rapid reoxygenation, or a lipid peroxide and the like. However, the details of the mechanism still remain unknown. In view of this, it is expected that if the active oxygen species is eliminated and the production of the lipid peroxide is inhibited, it will be possible to prevent the reduction of tissue function and the necrosis of the tissue and to thereby make the treatment and prophylaxis for various diseases possible.

Various agents for eliminating the active oxygen species or antioxidants have been proposed. However, these agents are not very effective for the treatment and prophylaxis of the diseases.

In Clinical Hemorheology, volume 15(1), (1945) pages 89-96 and volume 4 (1984) pages 15-28, Thrombosis Haemostas, volume 50(2), 1983, pages 604-609, US-A 384 9252 and FR-A 257 8745 medicaments are disclosed which contain batroxobin.

Hence, there is strong and urgent need for the development of a medicament for the treatment or prophylaxis of ischemia-reperfusion injury.

An object of the present invention is to provide use of batroxobin for the preparation of an effective medicament for the treatment or prophylaxis of ischemia-reperfusion injury.

The present invention provides use of batroxobin for the preparation of a pharmaceutical composition for the treatment or prophylaxis of ischemia-reperfusion injury which comprises an effective amount of batroxobin.

The present invention also provides use of batroxobin for the preparation of a pharmaceutical composition for the treatment or prophylaxis of ischemia-reperfusion injury in a patient with ischemiac myocardial disease which comprises an effective amount of batroxobin.

The present invention further provides use of batroxobin for the preparation of a pharmaceutical composition for the treatment or prophylaxis of ischemia-reperfusion injury in a patient with ischemiac cerebral disease which comprises an effective amount of batroxobin.

The inventors of this invention have discovered that batroxobin is effective or the treatment or prophylaxis of restenosis and arterial sclerosis after percutaneous transluminal angioplasty (PTA) and filed a patent application based on the discovery (USSN 08/555,451).

They have further conducted various studies with respect to the pharmaceutical effects of batroxobin. It was found out that batroxobin is effective in inhibiting cellular injury in a model of ischemia-reperfusion injury.

Batroxobin used in the present invention is a thrombin-like enzyme, preferably derived from snake (Bothrops atrox moojeni) venom and its formulation is commercially available under batroxobin formulation from Tobishi Pharmaceutical Co., Ltd.

The medicament of the present invention is used for the treatment or prophylaxis of the ischemia-reperfusion injury in myocardial disease, cerebrovascular disorder, kidney disease, liver disease, lung disease, pancreas disease and the like, or during organ transplantation.

Recently, percutaneous transluminal coronary revascularization (PTCR) and percutaneous transluminal coronary angioplasty (PTCA) have been widely conducted for decreasing myocardial necrosis after cardiac infarction. The medicament of the present invention is effective not only for myocardiopathy occurred when blood flow is restarted after such operations, but also for neurocyte disorders accompanying cerebral ischemic disorders when blood flow is restarted.

The components and their contents in 1 ml of batroxobin formulation [a thrombin-like enzyme derived from snake (Bothrops atrox moojeni) venom] are as follows:

| | |
|---|---|
| batroxobin (main component) | 10 BU |
| chlorobutanol (preservative) | 3 mg |
| gelatin hydrolyzate (stabilizer) | 0.1 mg |
| sodium chloride (isotonic agent) | 9 mg |
| distilled water for injection | to 1 ml |

The dosage of batroxobin employed in the present invention is dependent on the conditions of a patient. In general, the dosage is in the range of from 1 to 20 batroxobin units (hereinafter, abbreviated as BU) for an adult per day, although the dosage outside the above range can be used depending on the conditions of the patient.

Batroxobin may be suitably diluted and administered in the form of drip or injection, intravenously, intraarterially or topically. The batroxobin unit described herein is a unit representing an enzymatic activity of batroxobin such that the activity wherein the coagulation of plasma takes place in 19.0 ± 0.2 seconds when 0.1 ml of a batroxobin solution is added to 0.3 ml of standard human plasma containing citric acid at a temperature of 37°C is defined as 2 BU.

The acute toxicity test for batroxobin was conducted by intravenous administration to mice, rats, rabbits and dogs. The resulting LD₅₀ values (BU/kg) were as follows:

| kinds of animal | LD₅₀ value (BU/kg) |
|---|---|
| mice (ddY strain) | 192 ∼ 210 |
| rats (Wistar strain) | 105 ∼ 110 |
| rabbits (NW species) | > 300 |
| dogs (hybrid) | 190 ∼ 208 |

The present invention will hereunder be described in more detail with reference to the following Examples, but it should be understood that the present invention is not limited thereto.

### Example 1:

### Effects of batroxobin for the treatment or prophylaxis of ischemia-reperfusion injury in the myocardial reperfusion model in dogs.

### Experimental method:

30 mg/kg of sodium pentobarbital anesthetic was administered intravenously to each of 10 male and female dogs weighing 8 to 12 kg. After induction of anesthesia trachea intubation was conducted immediately followed by artificial respiration. After thoracotomy was conducted by excising a portion between the third and forth costas, the heart was exposed by breaking the pericardium. Ischemia was induced by desquamating the ramus desendens in front of the left coronary artery from ambient tissue and stopping the blood circulation temporarily by ligating it with an occluder. During the experiment, the stop of blood flow was recognized by monitoring the amount of coronary blood flow, the electro cardiogram and the left ventricle pressure. The reperfusion was then followed by removing the ligation. The evaluation of ischemia-reperfusion injury was conducted as described in detail as follows.

Blood circulation was re-started at 90 minutes after ligation and after 30 minutes, the heart beat was stopped by injecting immediately 10 ml of saturated potassium chloride solution. After washing the heart by perfusing retrogradely phosphate buffer from the arteria subclavia to the heart, the stenosis site was ligated again and then the non-ischemic region was stained by perfusing similarly 10 ml of 2% Evans blue solution in phosphate buffer. Thereafter, the heart was excised and out horizontally at a thickness of 1 cm, and the cut heart was placed in 1% TTC (Triphenyl Tetrazolium Chloride) and stained. Then, each area of Evans blue stained positive region (blue: non-ischemia area), Evans blue stained negative and TTC stained positive region (red: coronary ischemia healthy and normal area) and Evans blue stained negative and TTC stained negative region (white: cardiac infarction area) in the cross section of the cut heart was measured by planimeter. Then, the ischemia volume and the infarction volume were found from the area measured as described above and the thickness of each tissues whereby the percentage of infarction volume/ischemia volume ratio was calculated. In addition, in the group to which batroxobin was administered (n=5), 0.5 BU/kg of batroxobin was administered through the common jugular vein 30 minutes before starting the reperfusion of blood flow, and in the control group (n=5) , saline was administered similarly at the same volume.

### Results:

The percentage of infarction volume/ischemia volume ratio of the control group was 47 ± 10%. The percentage of infarction volume/ischemia volume ratio of the batroxobin treated group was 25 ± 5%. The results show that batroxobin inhibits significantly (p < 0.005) the extension of necrosis in the cardiac infarction area (Table 1). Therefore, it was found that the administration of batroxobin suppresses myocardial ischemia-reperfusion injury.

**Table 1**

| Specimen | Number | infarction volume/ischemia volume ratio(%) |
|---|---|---|
| Control | 5 | 47 ± 10 |
| Batroxobin | 5 | 25 ± 5 ** |

| | | |
|---|---|---|
| ** p < 0.005. | | |

### Example 2:

### Effects of batroxobin for the treatment and prophylaxis of ischemia-reperfusion injury in the cerebral ischemia-reperfusion model in rats.

### Experimental method:

18 Wistar male rats weighing about 200 to 250g were divided into three groups wherein each group consisted of 6 rats and referred to as a pseudo-operated group (sham group), a control group and a batroxobin treated group, respectively.

After the rat was anesthetized with 10% chloral hydrate (350 mg/kg), the rat was fixed in a stereotaxic operation apparatus and both sides alar ostia were exposed and both sides arteria vertebralis were shut down by the electro heat-coagulating method. After 24 hours, the cervical region median was excised under the same anesthetic condition as described above and on both sides the common carotid artery (CCA) were extracted. The extract was clamped by a clamp which does not result in damages for 30 minutes while monitoring brain waves.

The electrodes by which the brain wave were monitored were attached as follows: one electrode to the front nasal median site, and the other to the parietal region. At 30 minutes after the CCA was closed, the clamp was taken off again and cerebral perfusion was conducted for 6 hours. After the reperfusion was terminated, the head of the rat was cut off and the brain was delivered and fixed with formalin. After embedding the brain with paraffin, frontal cut pieces across the hippocumpus were prepared by cutting the brain into thin slices and then hematoxylin and eosin stain was applied to stain the samples. The degree of tissue injury was evaluated by observing the samples under an optical microscope to determine the number of normal pyramidal cells and injured pyramidal cells per 1 mm of length of hippocumpus CA1.

8 BU/kg of batroxobin was administered abdominally to the batroxobin treated group 30 minutes before clamping the CCA.

The same volume of saline was administered to the control group. In the pseudo-operated group, both sides alar ostia were exposed and after 24 hours, the CCA was exposed, but the same volume of saline was administered 30 minutes before exposing the CCA, without coagulating or clamping.

### Results:

Pseudo-operated group: No abnormal observations were found using an optical microscope. The survival rate of hippocumpus CA1 greater petrosal cells was 98.5 ± 1.6% (Table 2).
Control group: Observation under an optical microscope, revealed atrophia of the greater petrosal cells in the hippocumpus CA1 and the degree of the stain of cytoplasm was deep. In addition, cell nuclei were concentrated in the form of triangles and the spaces between the cells were increased. The survival rate of hippocumpus CA1 greater petrosal cells was 56.4±19.8% (Table 2).
Batroxobin treated group: Observation under an optical microscope revealed only slight atrophia of greater petrosal cells in the hippopcumpus CA1. The cytoplasm was only slightly stained. Cell nuclei and nucleoli were normal. The spaces between cells were also normal. The survival rate of hippocumpus CA1 greater petrosal cells was 87.3 ± 5.4%, which is significant as compared with the control group (p < 0.01) (Table 2).

Thus, the cell injury caused by cerebral ischemia-reperfusion was inhibited significantly by the batroxobin administration. Accordingly, it has been found that batroxobin effectively inhibits cerebral ischemia-reperfusion injury.

**Table 2**

| Effects of batroxobin on the survival rate of hippocumpus CA1 greater petrosal cells after cerebral ischemia-reperfusion | | |
|---|---|---|
| Group | the number of rats | survival rate (%) |
| Pseudo-operating | 6 | 98.5 ± 1.6 |
| Control | 6 | 56.4 ± 19.8 ¹⁾ |
| Batroxobin | 6 | 87.3 ± 5.4 ²⁾ |

| | | |
|---|---|---|
| The survival rate is expressed by the average ± S. D. ¹⁾ Comparison with pseudo-operated group, p < 0.01. | | |
| ²⁾ Comparison with control group, p < 0.01. | | |

## Claims

1. Use of a batroxobin for the preparation of a medicament for the treatment or prophylaxis of ischemia-reperfusion injury.

2. The use of claim 1, wherein the medicament is used for the treatment of an ischemiac myocardial disease.

3. The use of claim 1, wherein the medicament is used for the treatment of an ischemiac cerebral disease.

4. The use of claim 2, wherein batroboxin is contained in the range of from 1 to 20 BU.

5. The use of claim 3, wherein batroboxin is contained in the range of from 1 to 20 BU.

6. The use of claim 1, wherein batroxobin is a thrombin-like enzyme derived from the venome of Bothrops atrox moojeni.

## Patentansprüche

1. Verwendung eines Batroxobins zur Herstellung eines Arzneimittels für die Behandlung oder Prophylaxe einer Ischämie-Reperfusionsverletzung.

2. Verwendung nach Anspruch 1, wobei das Arzneimittel für die Behandlung einer ischämischen Herzmuskelerkrankung verwendet wird.

3. Verwendung nach Anspruch 1, wobei das Arzneimittel für die Behandlung einer ischämischen Gehirnerkrankung verwendet wird.

4. Verwendung nach Anspruch 2, wobei Batroxobin in einem Bereich von 1 bis 20 BU enthalten ist.

5. Verwendung nach Anspruch 3, wobei Batroxobin in einem Bereich von 1 bis 20 BU enthalten ist.

6. Verwendung nach Anspruch 1, wobei Batroxobin ein Thrombin-ähnliches Enzym ist, das vom Schlangengift von Bothrops atrox moojeni stammt.

## Revendications

1. Utilisation d'une batroxobine pour la préparation d'un médicament destiné au traitement ou la prophylaxie de lésions liées à une ischémie-reperfusion.

2. Utilisation selon la revendication 1, dans laquelle le médicament est utilisé pour le traitement d'une affection ischémique myocardique.

3. Utilisation selon la revendication 1, dans laquelle le médicament est utilisé pour le traitement d'une affection ischémique cérébrale.

4. Utilisation selon la revendication 2, dans laquelle la batroxobine est présente dans une gamme de 1 à 20 UB.

5. Utilisation selon la revendication 3, dans laquelle la batroxobine est présente dans une gamme de 1 à 20 UB.

6. Utilisation selon la revendication 1, dans laquelle la batroxobine est une enzyme de type thrombine provenant du venin de Bothrops atrox moojeni.
